(19) Europäisches Patentamt
European Patent Office
Office européen des brevets

(11) **EP 4 302 693 A1**

(12) **EUROPEAN PATENT APPLICATION**

(43) Date of publication:
**10.01.2024 Bulletin 2024/02**

(51) International Patent Classification (IPC):
**A61B 6/00** *(2006.01)*

(21) Application number: **22183591.1**

(52) Cooperative Patent Classification (CPC):
**A61B 6/5258; A61B 6/541**

(22) Date of filing: **07.07.2022**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(71) Applicant: **Koninklijke Philips N.V.**
**5656 AG Eindhoven (NL)**

(72) Inventors:
- **WENJIN, Wang**
  **Eindhoven (NL)**
- **FRERKING, Lena Christina**
  **Eindhoven (NL)**

- **SENEGAS, Julien Thomas**
  **5656AG Eindhoven (NL)**
- **WUELBERN, Jan Hendrik**
  **5656AG Eindhoven (NL)**
- **DEN BRINKER, Albertus Cornelis**
  **Eindhoven (NL)**
- **WEISS, Steffen**
  **Eindhoven (NL)**
- **PAPPOUS, Ioannis**
  **Eindhoven (NL)**

(74) Representative: **Philips Intellectual Property & Standards**
**High Tech Campus 52**
**5656 AG Eindhoven (NL)**

(54) **DETERMINING A TRIGGER SIGNAL FOR IMAGING**

(57)    A system (100) and method for determining a trigger signal for imaging with an imaging system (40) are provided. The method comprises the steps of:
- measuring (S10) a patient-specific characteristic of a patient (30). The patient-specific characteristic is measured when the patient (30) is located at a first position (51) relative to the imaging system (40);
- generating (S20) a patient-specific model based on the patient-specific characteristic;
- measuring (S30) a triggering waveform of the patient (30). The triggering waveform is measured when the patient (30) is located at a second position (52) relative to the imaging system (40). The second position (52) is different from the first position (51);
- determining (S40) the trigger signal based on the patient-specific model and the triggering waveform. The trigger signal is configured to trigger the imaging system (40) to acquire an image of the patient (30) in a time-resolved manner.

FIG. 5

**Description**

FIELD OF THE INVENTION

The invention relates to a system and method for determining a trigger signal for imaging with an imaging system.

BACKGROUND OF THE INVENTION

Electrocardiogram (ECG) and contact photoplethysmography (PPG), e.g. measured with a finger oximeter, have been used in standard magnetic resonance imaging (MRI) and computed tomography (CT) systems for cardiac triggering or gating of scans.

MRI typically uses triggering based on ECG measurements for scans which require synchronization to the cardiac pulse and/or heart phase. The ECG measurement holds the so-called R-peak, signaling the electrical activity starting the contraction of the heart muscle, i.e., the start of the systolic phase. Based on the R-peak, an MRI triggering sequence is started consisting of a preparation pulse (pre-pulse) and multiple acquisition windows. Such ECG based triggering may suffer from disadvantages like complex placement of the electrodes as well as multiple wires that can limit patient comfort as well as disturb imaging quality due to magnetic field interference.

Therefore, feasibility of cardiac-triggered MRI with camera-based PPG of the human face has been shown. A fully or semi-automated and contactless camera-PPG solution can significantly simplify the clinical MRI workflow because no contact sensors are required, and it may eliminate the interference of magnetic field to the signal.

However, compared to ECG-based triggering, a possible limitation of camera-PPG based triggering is that a patient-specific delay is present between the R-peak that is commonly used for cardiac triggering, and the trigger derived from PPG signals. This delay is caused by the pulse transit times from the heart to the peripheral skin site and may lead to inaccurate triggering unless compensated for.

The publication US2017055934A1 discloses a method and system for determining a trigger signal. The method for determining a trigger signal for an imaging device is based on receiving a film of a surface of a first body part of a patient. In an embodiment, image values in a region in the images of the film are averaged. The noise of the image values in the region is reduced by way of the averaging. The film is transformed into a temporal signal series on the basis of the averaging. The transformation takes place in such a manner that the temporal series is a measure of the timing pattern of the blood circulation in the area.

Camera-based PPG requires a certain amount processing in order to achieve robust trigger detection. This may lead to costly delays in the workflow before actual triggered scanning can commence.

Hence, there is a need for improved speed and accuracy of triggered imaging.

SUMMARY OF THE INVENTION

It is an object of the invention to provide a method and system to improve the speed and accuracy of triggered imaging.

The invention is defined by the independent claims. Advantageous embodiments are defined in the dependent claims.

According to a first aspect of the invention there is provided a method for determining a trigger signal for imaging with an imaging system. The method comprises the steps of:

- measuring a patient-specific characteristic of a patient. The patient-specific characteristic is measured when the patient is located at a first position relative to the imaging system;
- generating a patient-specific model based on the patient-specific characteristic;
- measuring a triggering waveform of the patient. The triggering waveform is measured when the patient is located at a second position relative to the imaging system. The second position is different from the first position;
- determining the trigger signal based on the patient-specific model and the triggering waveform. The trigger signal is configured to trigger the imaging system to acquire an image of the patient in a time-resolved manner.

The patient-specific characteristic is a measured characteristic related to the physiology or physical characteristics of the patient, such as, but not limited to, heart rate, respiration, oxygenation, blood pressure, cardiac cycle, temperature, weight, height, body-mass-index etc. The patient-specific characteristic may be a constant, one or several measurement points, amplitude, frequency, phase, shape, signal-to-noise level etc. of a physiological measurement. This characteristic or combinations of characteristics can be used to model parameters that are specific for the patient in a patient-specific model. Similarly, the triggering waveform is a time-resolved measurement of a physiological parameter of the patient, such as, but not limited to, heart rate, respiration, oxygenation, cardiac cycle, temperature etc. The trigger signal directly or indirectly triggers acquisition of an image of the patient with the imaging system at a moment in time.

The proposed method may be advantageous for speed and accuracy of the imaging triggering workflow since one or multiple patient-specific characteristics can be measured, and generation of a patient-specific model from such characteristics can be initiated, already before the patient is in the second position where the triggering waveform is measured. The second position may also be the position of the patient when the imaging system is triggered to acquire an image of the patient. In this way, the combination of patient-specific information and the measured triggering waveform may form the basis for accurate imaging triggering without causing unnecessary delays to the workflow.

In an embodiment of the invention, in the first position a patient anatomy of interest is located substantially outside of a bore of the imaging system, and in the second position said patient anatomy of interest is located substantially inside the bore of the imaging system.

The patient anatomy of interest is a part of the patient where triggering is determined and/or the body part that is imaged by the imaging system. By measuring patient-specific characteristics when the patient anatomy of interest is outside of the bore of the imaging system, such as e.g. during a patient preparation phase, patient time inside the bore can be shortened. In this way the total workflow time may be shortened, and patient comfort may be increased. The bore of an imaging system may be e.g. the bore of a computed tomography (CT) system or a magnetic resonance imaging (MRI) system or a molecular imaging system, like positron emission tomography (PET) or single photon emission computed tomography (SPECT), or any combination of such systems.

In an embodiment of the invention, the triggering waveform comprises a waveform derived from photoplethysmography (PPG), and the step of measuring the triggering waveform comprises a measurement with a sensor, preferably a camera.

A measurement of the triggering waveform with e.g. camera-based PPG, instead of multiple electrocardiogram (ECG) sensors placed in contact with the patient may have multiple advantages, such as automation or simplification of the workflow, avoidance of magnetic field interference of the ECG signal etc.

The sensor is preferably e.g. a 2D or 3D camera sensor or similar, configured to contactless measure PPG parameters from visible light or infrared light or light of other wavelengths. However, other sensor technologies are also conceivable to measure a waveform derived from PPG, such transmissive or reflective PPG sensors in close proximity to or contact with the patient, contactless measurements with RADAR or LIDAR, etc.

In an embodiment of the invention, the patient-specific characteristic comprises a characteristic derived from PPG, and the step of measuring the patient-specific characteristic comprises a measurement with a sensor, preferably a camera.

Similarly to the triggering waveform, a measurement of patient-specific parameters with e.g. camera-based PPG, alone or in combination with other measurements, may speed up and simplify the imaging and/or patient preparation workflow.

The sensor used to measure a patient-specific characteristic, comprising a characteristic derived from PPG, may be the same sensor as the sensor used to measure the triggering waveform. The sensor may also be a separate sensor of the same, similar, or different type or functionality as the sensor used to measure the triggering waveform. By way of example, both sensors may e.g. be sensors configured to measure camera-based PPG signals.

In an embodiment of the invention, the patient-specific model comprises a patient-specific PPG waveform profile.

Modelling of a patient-specific model comprising a PPG waveform profile, such as a template morphology and/or amplitude of the waveform, is advantageous for fast and accurate imaging triggering. This may be of particular importance when the triggering waveform comprises a waveform derived from PPG.

In an embodiment of the invention, the patient-specific model comprises a patient-specific pulse transit time.

Similarly to the patient specific-waveform profile, information about a patient-specific pulse transit time may be advantageous for fast and accurate imaging triggering.

In an embodiment of the invention, the patient-specific model comprises a patient-specific reliability factor of a camera-based PPG parameter.

The patient-specific reliability factor may provide an indication of the reliability, quality and/or robustness of camera-based PPG for the specific patient and/or circumstances. If e.g. the reliability is low, additional or alternative measurements to camera-based PPG may be required for accurate triggering based on the triggering waveform. Having such information at an early stage may save bore time and/or avoid that sets of image acquisitions need to be repeated.

In an embodiment of the invention, the step of determining the trigger signal comprises calculating multiple PPG markers, wherein the PPG markers are derived from the triggering waveform and/or the patient-specific PPG waveform profile, and wherein the PPG markers are configured to predict an R-peak.

PPG markers are specific PPG features of the PPG waveform, such as inter-beat interval, waveform features, phase etc. Calculating multiple PPG markers may be advantageous, since the PPG markers may improve accuracy and/or robustness when predicting an R-peak from e.g. camera-based PPG.

In an embodiment of the invention, the PPG markers as mentioned above comprise a diastolic phase PPG marker and a flush phase PPG marker.

In an embodiment of the invention, the method further comprises the steps of:

- deriving a timing parameter from the triggering waveform and/or the patient-specific model, and
- scanning the patient triggered by the trigger signal, wherein scanning is configured to generate imaging scan data.

In an embodiment of the invention, the method further comprises an image formation step, wherein the image formation step forms an image of the patient based on processing of the imaging scan data and the timing parameter.

This may advantageous, since using a timing parameter from the triggering waveform and/or the patient-specific model can help to improve the quality and/or speed of image formation.

According to another aspect of the invention, there is provided a computer program element, which, when being executed by at least one processing unit, is adapted to cause the processing unit to perform a for the processing unit any computer-executable method step according to any of the methods described above.

According to another aspect of the invention, there is provided a computer readable medium having stored thereon the above-mentioned computer program element.

According to a second aspect of the invention, there is provided a system for determining a trigger signal for imaging a patient with an imaging system, the system comprising:

- a patient-specific characteristic sensor configured to measure a patient-specific characteristic of a patient when the patient is located at a first position relative to the imaging system,
- a triggering waveform sensor configured to measure a triggering waveform of the patient when the patient is located at a second position relative to the imaging system, and
- a processing unit configured to receive the patient-specific characteristic, generate a patient-specific model based on the patient-specific characteristic, receive the triggering waveform, and determine an imaging trigger signal based on the patient-specific model and the triggering waveform.

In an embodiment of the invention, at least one of the patient-specific characteristic sensor and/or the triggering waveform sensor is a sensor, preferably a camera, and the sensor is configured to measure a sensor-based PPG signal.

According to another aspect of the invention, there is provided an imaging system comprising the system for determining a trigger signal, wherein the imaging system is preferably a computed tomography system or a magnetic resonance imaging system.

These and other aspects of the invention will be apparent from and elucidated with reference to the embodiments described hereinafter.

BRIEF DESCRIPTION OF THE DRAWINGS

Fig. 1 illustrates a schematic of a system for determining a trigger signal, in accordance with an embodiment of the invention.
Fig. 2 illustrates a schematic of measured and processed signals according to an embodiment of the invention.
Fig. 3 shows palm and face camera-PPG signals used to determine a patient-specific model according to an embodiment of the invention.
Fig. 4 illustrates determination of multiple PPG markers according to an embodiment of the invention.
Fig. 5 shows a flowchart representing a method of determining a trigger signal in accordance with an embodiment of the invention.
Fig. 6 shows a flowchart representing a method of determining a trigger signal to initiate a scan in accordance with an embodiment of the invention.
Fig. 7 shows a flowchart representing a method of image formation in accordance with an embodiment of the invention.

DESCRIPTION OF EMBODIMENTS

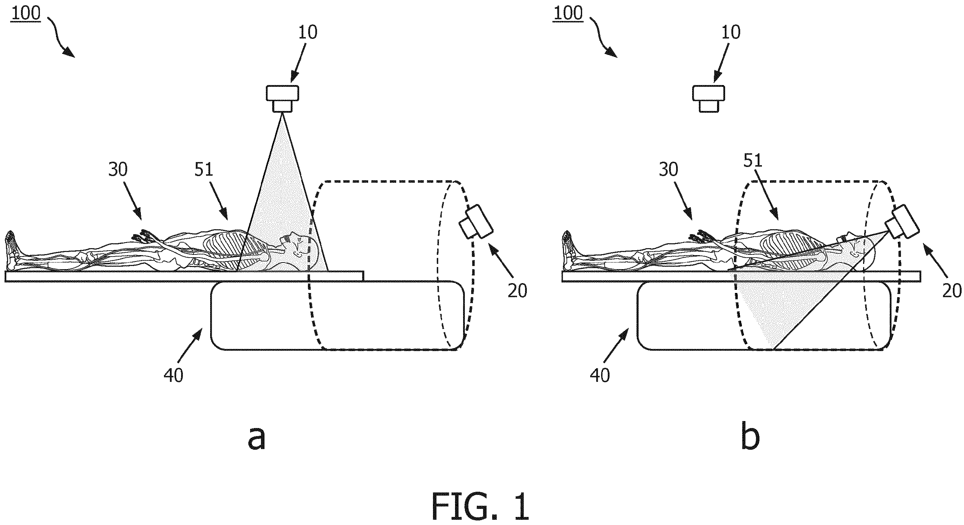

A schematic of a system 100 for determining a trigger signal, in accordance with embodiments of the invention, is illustrated in Fig. 1. A patient 30 is positioned on the table of an imaging system 40. In this example it is proposed to use an out-bore camera sensor 10 to measure patient-specific characteristics that can be used to model the patient-specific physiology during the preparation phase, before sending the patient into the bore for triggered scanning using the in-bore camera sensor 20.

Many imaging system environments already offer a ceiling-mounted camera that allows for monitoring the patient during the preparation phase, before moving the table into the bore. Such camera sensors can therefore be further used for e.g. remote PPG analysis. The patient-specific characteristic sensor 10 and the triggering waveform sensor 20 are connected, via a wired or wireless connection, to a processing unit (not shown in the figure).

In the example illustrated in Fig 1. the two sensors are separate camera sensors at different locations. However, it is also possible that the patient-specific characteristic sensor 10 and the triggering waveform sensor 20 is the same sensor and/or located at the same location. Such as e.g. a camera-based sensor with a field of view both inside and outside the bore of the imaging system 40.

In Fig. 1a, the patient 30 is in a first position 51 during the preparation phase. In the first position 51, the patient anatomy of interest, such as the chest or the head or another body part, is substantially outside the bore of the imaging system 40. It should be noted that although Fig. 1a illustrates the patient on a table in connection to the imaging system, it is also conceivable that the first position 51 is further away from the imaging system, such as in another room used for preparation.

During the phase illustrated in Fig. 1a, when the patient is in a first position 51, patient-specific characteristics are measured with the camera sensor 10. The measurement may be combined with measurements by additional sensors remotely or in contact with the patient. Examples of such measured characteristics include, but are not limited to, characteristics derived from PPG, such as pulse waveform, heart rate, respiration rate, blood pressure, the relationships between pulsatile features and ECG R-peak, PPG peak detection rate etc. Based on one or multiple patient-specific characteristics, a patient-specific model can be created at the processing unit. Non-limiting examples of such a patient-specific model that can be created from patient-specific characteristics measured with a camera sensor 10, include a patient-specific PPG waveform profile, a patient-specific pulse transit time or a patient-specific reliability factor of a camera-based PPG parameter.

In Fig. 1b, the patient 30 is in a second position 52 during the scanning phase. In the second position 52, the patient anatomy of interest is substantially inside the bore of the imaging system 40. In the second position 52, a triggering waveform is measured with the camera sensor 20, in this example an in-bore camera. The triggering waveform may be derived from a camera-based PPG measurement with the camera sensor 20. The processing unit receives the triggering waveform and determines an imaging trigger signal. The trigger signal is configured to trigger the imaging system 40 to acquire an image of the patient 30 in a time-resolved manner.

Several potential advantages can be envisioned from such a system as illustrated in Fig. 1, where the patient-specific characteristics can be measured already during the patient preparation phase and/or at least before the patient 30 is moved further into the bore of the imaging system 40. Examples include;

With knowledge of patient-specific PPG models, such as PPG waveform morphology, PPG amplitude, pulse transit time delays etc., camera-based accurate and fast timing of e.g. the MRI and CT acquisition windows is simplified.

Images of MRI cardiac cine movies are commonly annotated with time stamps relative to the R-peak, which is defined as time zero. This is possible with prior knowledge of the delays.

MRI cardiac cine movies are typically presented for diagnosis and comparison such that they start with the time of the R-peak. This is possible with prior knowledge of the delays.

Functional evaluation of cardiac images as for ejection fraction and wall-motion may be simplified and/or more accurate with knowledge about timing relative to the R-peak.

Triggering with a low delay between R-peak and trigger requires a patient specific PPG template and therefore some initial calibration time to initialize this model. When such calibration can be made in advance by the processing unit, without relying on an in-bore measurement, expensive in-bore time can be saved.

When camera-based PPG signal quality can be evaluated in advance, to calculate a patient-specific reliability factor of a camera-based PPG parameter, this can have significant positive effects on the workflow time. E.g. in an example of triggered MRI, the workflow would be prolonged drastically if the patient is already inside the bore and the in-bore camera 20 detects a patient-specific insufficient PPG signal quality. In that case the patient 30 would have to be taken out again, MR coils removed, ECG-electrodes applied, MR coils applied again, and then the patient 30 would have to be repositioned in the bore.

In the example illustrated in Fig 1, the out-bore camera 10 is positioned on top (or side) of the patient 30 lying on the table and measuring the physiological signals from the patient skin (e.g. the chest or face area). The physiological signal may be a PPG signal, but it can also include other physiological signals. In some cases an ECG sensor is also used. The ECG signal, measured with the ECG sensor, may e.g. be synchronously measured with the camera-PPG signal.

Current ECG-detection for MRI triggering is typically based on at least four electrodes on the bare chest. At least four electrodes are typically required for robust R-peak detection inside the bore, because here the ECG signal may be corrupted due to interference with MR fields. For synchronous measurement of ECG with PPG outside of the bore for the purpose of measuring patient-specific characteristics and initialization of calculating a patient-specific model, it is possible to apply only two ECG-electrodes. The electrodes may be applied e.g. at the wrists or hands of the patient 30. Such an ECG set-up may simplify the workflow tremendously. Furthermore, in such cases where the electrodes are removed before the patient enters the bore, since in-bore triggering waveform is measured with e.g. camera-PPG, the ECG electrodes, leads and detection circuitry do not have to be made MR-compatible. It can be envisioned that in some cases the ECG electrodes may just be simple handles or conductive fields at both sides of the patient support that may be grabbed for a few seconds by the patient for as long as data is required to calibrate the model.

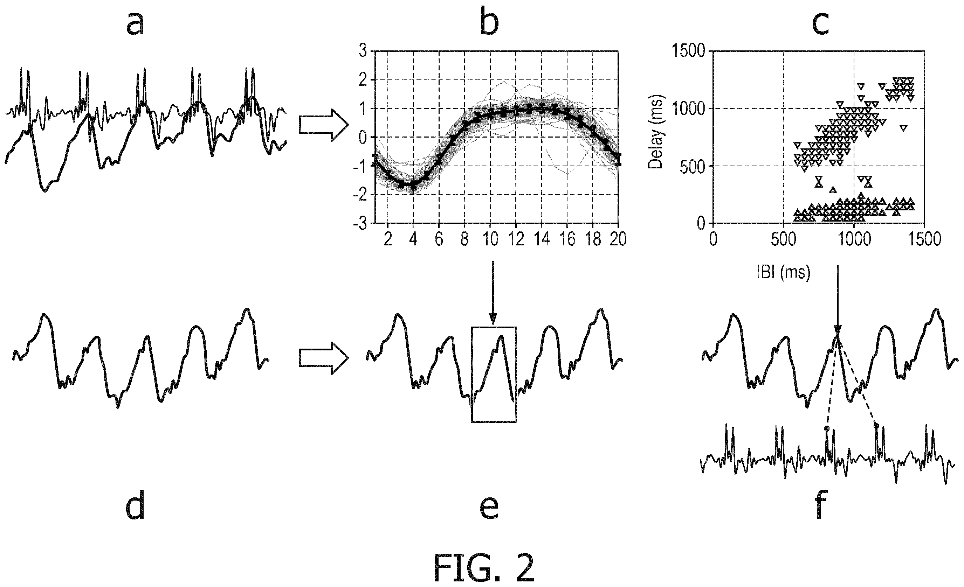

Fig. 2 illustrates an example of the relation between the patient-specific characteristics measured when the patient 30 is at the first position 51, the patient-specific model, and the triggering signal measured when the patient 30 is at the second position 52.

Fig. 2a schematically shows the measured patient-specific characteristics when the patient 30 is at the first position 51. In this example, a signal derived from PPG is measured with the patient-specific characteristics sensor 10, such as a camera PPG sensor. In addition, an ECG signal is measured with an ECG sensor. The synchronized camera-PPG derived signal and ECG signal can be used to calculate patient specific models, as illustrated in Fig. 2b and Fig 2c.

Fig. 2b illustrates a created PPG waveform profile that can be used as a patient-specific PPG template to enable fast triggering. The PPG waveform profile is a robust PPG cardiac cycle averaged from multiple PPG cycles, e.g. as measured by face or chest camera-PPG in Fig 2a. Since this model is based on the patient-specific characteristic measured when the patient is at the first position 51, the model can be initialized before the patient moves further into the bore of the imaging system 40.

Similarly, Fig 2c shows a created R-peak regression model that models the relationship between the camera-PPG features and ECG R-peaks measured. The input to the model comes from the patient-specific characteristics measured at the first position 51. The model may associate specific PPG features (e.g. inter-beat interval, waveform features, phase), so called PPG markers, with the pulse transit time between R-peak and systolic valley of a camera-PPG signal. Such a regression model can therefore be used to predict/regress R-peak location from a camera-based PPG waveform.

Fig. 2d illustrates a triggering waveform that is measured when the patient 30 has moved further into the bore of the imaging system 40. In this example, the triggering waveform is derived only from camera-based PPG measurements when the patient is at the second position 52. Thus, no ECG is required inside the imaging system to measure the triggering waveform.

As shown in Fig. 2e, the previously created PPG waveform profile (Fig. 2b) can be used to quickly identify PPG cycles in the triggering waveform, which may be used for trigger detection. Since building of the model was already initialized earlier in the workflow, fast triggering can be enabled.

Similarly, as shown in Fig. 2f, the pre-built R-peak regression model (Fig 2c) can be used to enable accurate triggering by predicting ECG R-peaks from the camera-based triggering waveform.

A trigger signal is generated based on the identified cardiac cycles and R-peaks, such that the imaging system is triggered to acquire images of the patient.

In the example illustrated in Fig 2., the patient-specific characteristic used to model the patient specific pulse transit time is measured in the patient preparation phase by the combination of camera-based PPG and ECG. Alternatively, the pulse transit time and related parameters can also be modelled from PPG measurements such as multi-site PPG imaging without the need for ECG.

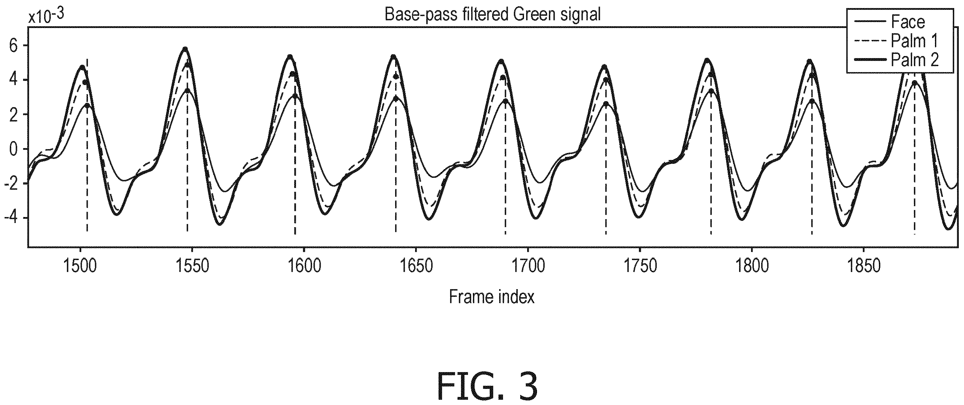

Fig. 3 illustrates multi-site PPG imaging, where the PPG signals are acquired simultaneously from the face and palms of the patient with a ceiling camera, which can be the patient-specific characteristics sensor 10. Information about simultaneous signals between face and palms can be used to estimate a surrogate transit time. Such a surrogate transit time can in turn be used to improve the accuracy of R-peak regression from PPG signals.

Additional parameters like patient height, age, body-mass index etc. may also be used to further improve the models.

In order to improve the robustness of PPG marker detection from the triggering waveform, multiple PPG markers can be generated in combination with patient-specific PPG waveform profiles. In this way one marker may predict the next and thereby improve the robustness of the model.

Instead of creating one single patient-specific PPG waveform profile, it is also possible to create a multitude of profiles associated with specific cardiac cycle durations or for ranges of cardiac cycle durations. Creating different profiles per ranges of cycle duration may be motivated by the fact that the time curves of cardiac beat and related physiological quantities as arterial blood velocity and PPG signal do not simply scale linearly if the heart rate changes. Instead, some parts of the cardiac cycle are extended in time (as typically the resting phase in diastole) and some parts remain more or less unchanged (as the cardiac contraction phase).

In each waveform profile, one or more PPG markers can be defined. Of particular relevance are the zero-crossings in this signal. The negative-going zero-crossing is associated with the highest blood volume change at the observation point, e.g. forehead with camera-PPG, in response to a contraction of the heart. The positive-going zero-crossing is associated with the diastolic phase and typically precedes the ECG R-peak. A plurality of markers may by generated from a single waveform profile thus enabling tracking the current phase of the PPG signal in the cardiac cycle.

Detection of PPG markers can e.g. be done as follows. The waveform profile can be shifted so that it starts and stops with a zero-crossing. In this way two waveform profiles are created, each with a full cardiac waveform: one beginning and ending with a positive-going zero-crossing and the second one beginning and ending with a negative-going zero-crossing.

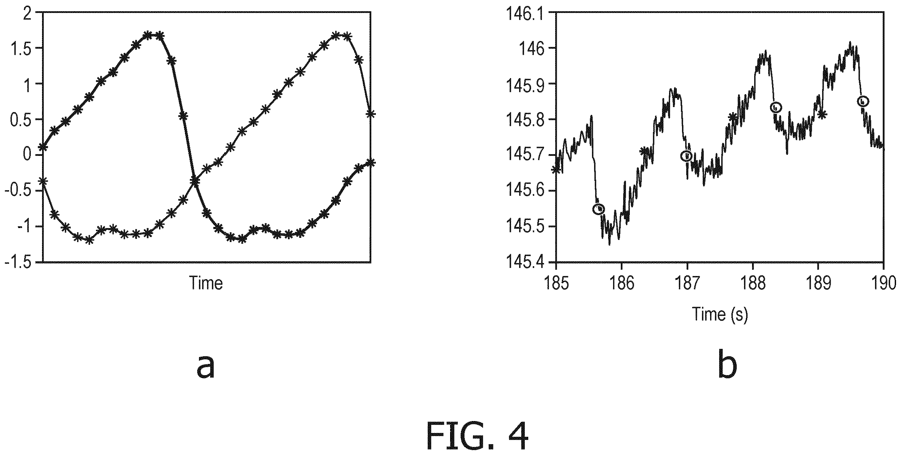

Both waveforms are shown in Fig. 4a, where the PPG waveform profile is stretched or time-warped to cover 33 samples. The notion is to check if the latest received sample of incoming PPG waveform corresponds to the last sample in these prototypes. If so, the corresponding marker is generated.

The patient-specific PPG signal may be decomposed using a patient-specific model. The model describes the waveform as being composed of three parts: the PPG waveform profile, a low frequency (LF) signal component and a noise component. The LF signal component can be modelled as a low-order polynomial. More formally, let the discrete-time PPG signal segment be denoted as s(n), n=[1,...,N], the prototype as p(n), the LF signal component as b(n), then the signal is described as

$$s = \alpha\, p + b + e,$$

with e(n) the error signal describing noise (e.g., measurement noise) and disturbances, and with $\alpha$ being a scale factor. The signal component b captures the signal drifting in the PPG and PPG variation associated with respiration (and thus of lower frequency than the heart rate). Various ways to arrive at such a decomposition are known, e.g., a least-squares fitting procedure may be used to attain said decomposition, where windowing may be used to stress the importance of certain parts of the segment in the fit.

The decomposition signals can be analyzed for their strength, e.g., in terms of energy. A good fit is attained if the energy associated with the PPG component p is significantly larger than that associated with e. Further evidence supporting the generation of the marker would be that the coefficient $\alpha$ is positive. Further evidence would be that when considering the next segment consisting of one simple shifted input signal, the coefficient $\alpha$ has become lower, or the balance between the energies in ($\alpha$ p) and e has become less favorable. In these or other ways, a decision may be taken that the marker has occurred.

Since the length of the current cardiac cycle is a priori unknown, the system operates at various lengths. Thus, the detection has an extra dimension, namely that along the scale (cardiac cycle length). The various cues for the existence of the marker also have to be seen across this extra dimension. A hard decision may be taken based on thresholding, more statistical-like approaches may be used, or neural nets may be trained to create the final decision mechanism

Examples of multiple generated PPG markers to improve R-peak prediction are illustrated in Fig 4b. More specifically, prediction can be improved by choosing at least one marker preceding but close to the R-peak and at least a second marker indicating that an R-peak has occurred, e.g., by observing blood volume change induced by the induced heart contraction. The PPG markers are in this case generated at the diastolic phase and at or near the moment of highest blood volume change, the flush phase. The diastolic phase PPG marker (asterisks in Fig 4b) typically comes just before the R-peak and the flush phase PPG marker (circles in Fig 4b) typically just after. The combination of the two therefore improves timely prediction of the R-peak.

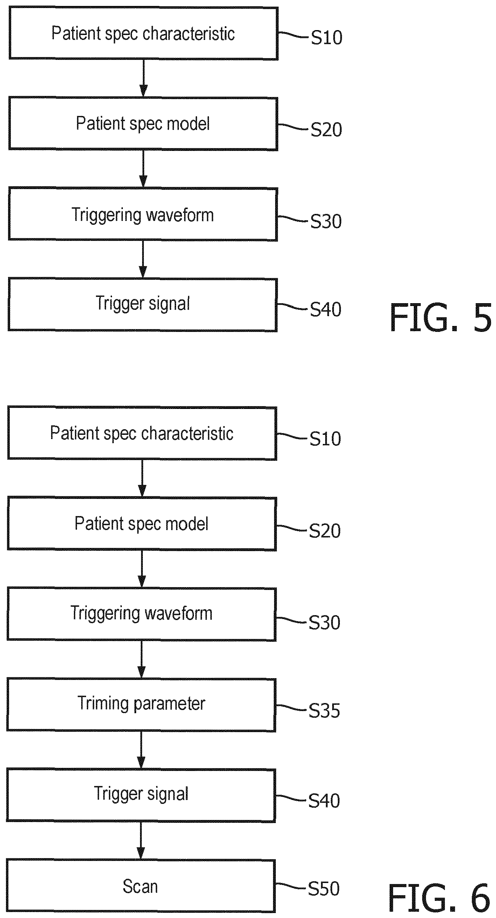

In the above description, several embodiments of the invention are elaborated on. An aspect of the invention is also illustrated by Fig. 5, showing a flowchart representing a method of determining a trigger signal. The method comprises the steps of:

- measuring S10 a patient-specific characteristic of a patient. The patient-specific characteristic is measured when the patient is located at a first position relative to the imaging system;
- generating S20 a patient-specific model based on the patient-specific characteristic;
- measuring S30 a triggering waveform of the patient. The triggering waveform is measured when the patient is located at a second position relative to the imaging system. The second position is different from the first position;
- determining S40 the trigger signal based on the patient-specific model and the triggering waveform. The trigger signal is configured to trigger the imaging system to acquire an image of the patient in a time-resolved manner.

Similarly, Fig. 6 shows a flowchart representing a method of determining a trigger signal to initiate a scan in accordance with an embodiment of the invention. In this case, the method in addition to what is shown in Fig. 5 also includes the steps of deriving S35 a timing parameter from the triggering waveform and/or the patient-specific model, and scanning S50 the patient triggered by the trigger signal, wherein scanning S50 is configured to generate imaging scan data.

Besides achieving a more robust triggering from the measured triggering waveform, patient-specific models, e.g. including PPG markers, may contain timing parameters that can be further leveraged during the imaging workflow. An image formation process S60, where scan data is combined into an image or video sequence, may use such timing parameters as extra information to control the image formation process. E.g. by weighting or as a compensation factor for the data obtained at different data acquisition windows.

E.g. in MRI, knowledge about timing parameters as the distance between pre-pulse generation and data acquisition timing can be used to improve image formation. Alternatively, the trigger mechanism can be configured to keep stricter requirements on constant pre-pulse to acquisition window distance. In this case the information of the occurrence of each MR sequence event, such as the motion state of the myocardium, can be used during the image formation process.

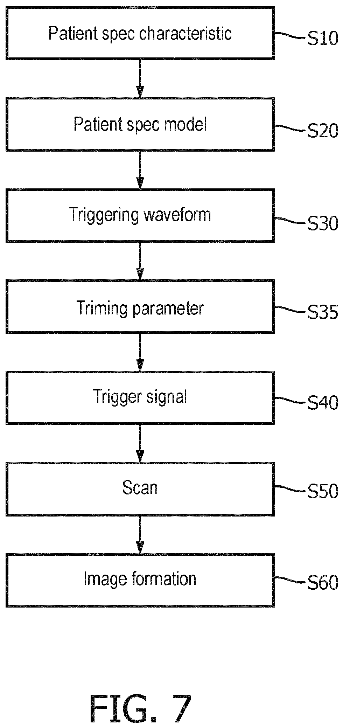

Fig. 7 illustrates an embodiment of the invention, where the method as shown in Fig. 6 further comprises an image formation step S60, wherein the image formation step S60 forms an image of the patient based on processing of the imaging scan data and the timing parameter.

It should be noted that the above-mentioned embodiments illustrate rather than limit the invention, and that those skilled in the art will be able to design many alternative embodiments without departing from the scope of the appended claims. In the claims, any reference signs placed between parentheses shall not be construed as limiting the claim. The word "comprising" does not exclude the presence of elements or steps other than those listed in a claim. The word "a" or "an" preceding an element does not exclude the presence of a plurality of such elements. The invention may be implemented by means of hardware comprising several distinct elements, and/or by means of a suitably programmed processor. In the device claim enumerating several means, several of these means may be embodied by one and the same item of hardware. Measures recited in mutually different dependent claims may advantageously be used in combination.

Reference signs

| 100 | system for determining a trigger signal |
| 10 | patient-specific characteristic sensor |
| 20 | triggering waveform sensor |
| 30 | patient |
| 40 | imaging system |
| 51 | first position |
| 52 | second position |
| S10 | measuring a patient-specific characteristic |
| S20 | generating a patient-specific model |
| S30 | measuring a triggering waveform |
| S35 | deriving a timing parameter |
| S40 | determining a trigger signal |
| S50 | scanning the patient |
| S60 | image formation process |

**Claims**

1. A method for determining a trigger signal for imaging with an imaging system (40), the method comprising the steps of:

   - measuring (S10) a patient-specific characteristic of a patient (30), wherein the patient-specific characteristic is measured when the patient (30) is located at a first position (51) relative to the imaging system (40),
   - generating (S20) a patient-specific model based on the patient-specific characteristic,
   - measuring (S30) a triggering waveform of the patient (30), wherein the triggering waveform is measured when the patient is located at a second position (52) relative to the imaging system (40), wherein said second position (52) is different from the first position (51), and
   - determining (S40) the trigger signal based on the patient-specific model and the triggering waveform, wherein the trigger signal is configured to trigger the imaging system (40) to acquire an image of the patient (30) in a time-resolved manner.

2. The method of claim 1, wherein in the first position (51) a patient anatomy of interest is located substantially outside of a bore of the imaging system (40), and wherein in the second position (52) said patient anatomy of interest is located substantially inside the bore of the imaging system (40).

3. The method according to claim 1 or 2, wherein the triggering waveform comprises a waveform derived from photoplethysmography (PPG), and wherein the step of measuring the triggering waveform comprises a measurement with a sensor, preferably a camera.

4. The method according to any of the preceding claims, wherein the patient-specific characteristic comprises a characteristic derived from PPG, and wherein the step of measuring the patient-specific characteristic comprises a measurement with a sensor, preferably a camera.

5. The method according to any of the preceding claims, wherein the patient-specific model comprises a patient-specific PPG waveform profile.

6. The method according to any of the preceding claims, wherein the patient-specific model comprises a patient-specific pulse transit time.

7. The method according to any of the preceding claims, wherein the patient-specific model comprises a patient-specific reliability factor of a camera-based PPG parameter.

8. The method according to claim 5, wherein the step of determining the trigger signal comprises calculating multiple PPG markers, wherein the PPG markers are derived from the triggering waveform and/or the patient-specific PPG waveform profile, and wherein the PPG markers are configured to predict an R-peak.

9. The method according to claim 8, wherein the PPG markers comprise a diastolic phase PPG marker and a flush phase PPG marker.

10. A method according to any of the preceding claims, the method further comprising the steps of:

    - deriving (S35) a timing parameter from the triggering waveform and/or the patient-specific model,
    - scanning (S50) the patient triggered by the trigger signal, wherein scanning is configured to generate imaging scan data, and
    - image formation (S60), wherein an image of the patient (30) is formed based on processing of the imaging scan data and the timing parameter.

11. A computer program element, which, when being executed by at least one processing unit, is adapted to cause the processing unit to perform a for the processing unit any computer-executable method step according to any of the preceding claims.

12. A computer readable medium having stored thereon the program element of claim 11.

**13.** A system (100) for determining a trigger signal for imaging a patient (30) with an imaging system (40), the system (100) comprising:

- a patient-specific characteristic sensor (10) configured to measure a patient-specific characteristic of a patient (30) when the patient (30) is located at a first position (51) relative to the imaging system (40),
- a triggering waveform sensor (20) configured to measure a triggering waveform of the patient (30) when the patient (30) is located at a second position (52) relative to the imaging system (40), and
- a processing unit configured to receive the patient-specific characteristic, generate a patient-specific model based on the patient-specific characteristic, receive the triggering waveform, and determine an imaging trigger signal based on the patient-specific model and the triggering waveform.

**14.** An imaging system (40) comprising the system (100) for determining a trigger signal, according to claim 13, wherein the imaging system (40) is preferably a computed tomography system or a magnetic resonance imaging system.

a

b

FIG. 1

EP 4 302 693 A1

FIG. 2

FIG. 3

FIG. 4

```
┌─────────────────────────────┐
│  Patient spec characteristic │──S10
└─────────────────────────────┘
              │
              ▼
┌─────────────────────────────┐
│     Patient spec model       │──S20
└─────────────────────────────┘
              │
              ▼
┌─────────────────────────────┐
│     Triggering waveform      │──S30
└─────────────────────────────┘
              │
              ▼
┌─────────────────────────────┐
│        Trigger signal        │──S40
└─────────────────────────────┘
```

# FIG. 5

```
┌─────────────────────────────┐
│  Patient spec characteristic │──S10
└─────────────────────────────┘
              │
              ▼
┌─────────────────────────────┐
│     Patient spec model       │──S20
└─────────────────────────────┘
              │
              ▼
┌─────────────────────────────┐
│     Triggering waveform      │──S30
└─────────────────────────────┘
              │
              ▼
┌─────────────────────────────┐
│      Triming parameter       │──S35
└─────────────────────────────┘
              │
              ▼
┌─────────────────────────────┐
│        Trigger signal        │──S40
└─────────────────────────────┘
              │
              ▼
┌─────────────────────────────┐
│            Scan              │──S50
└─────────────────────────────┘
```

# FIG. 6

```
┌─────────────────────────────────┐
│   Patient spec characteristic   │──S10
└─────────────────────────────────┘
                 │
                 ▼
┌─────────────────────────────────┐
│        Patient spec model       │──S20
└─────────────────────────────────┘
                 │
                 ▼
┌─────────────────────────────────┐
│        Triggering waveform      │──S30
└─────────────────────────────────┘
                 │
                 ▼
┌─────────────────────────────────┐
│         Triming parameter       │──S35
└─────────────────────────────────┘
                 │
                 ▼
┌─────────────────────────────────┐
│          Trigger signal         │──S40
└─────────────────────────────────┘
                 │
                 ▼
┌─────────────────────────────────┐
│               Scan              │──S50
└─────────────────────────────────┘
                 │
                 ▼
┌─────────────────────────────────┐
│         Image formation         │──S60
└─────────────────────────────────┘
```

# FIG. 7

Europäisches
Patentamt

European
Patent Office

Office européen
des brevets

## EUROPEAN SEARCH REPORT

**Application Number**

**EP 22 18 3591**

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | US 2021/353244 A1 (KIELY JANID PATRICIA BLANCO [US]) 18 November 2021 (2021-11-18) * paragraphs [0206] - [0208], [0210] * ----- | 1-14 | INV.<br>A61B6/00 |
| | | | **TECHNICAL FIELDS SEARCHED (IPC)**<br><br>A61B |

The present search report has been drawn up for all claims

| Place of search | Date of completion of the search | Examiner |
|---|---|---|
| Munich | 23 November 2022 | Anscombe, Marcel |

EPO FORM 1503 03.82 (P04C01)

## ANNEX TO THE EUROPEAN SEARCH REPORT
## ON EUROPEAN PATENT APPLICATION NO.

EP 22 18 3591

This annex lists the patent family members relating to the patent documents cited in the above-mentioned European search report.
The members are as contained in the European Patent Office EDP file on
The European Patent Office is in no way liable for these particulars which are merely given for the purpose of information.

23-11-2022

| Patent document cited in search report | | Publication date | Patent family member(s) | | Publication date |
|---|---|---|---|---|---|
| US 2021353244 | A1 | 18-11-2021 | AU 2019477068 | A1 | 02-06-2022 |
| | | | CA 3161947 | A1 | 10-06-2021 |
| | | | CN 114980970 | A | 30-08-2022 |
| | | | EP 4069354 | A1 | 12-10-2022 |
| | | | KR 20220124166 | A | 13-09-2022 |
| | | | TW 202122038 | A | 16-06-2021 |
| | | | US 2021353244 | A1 | 18-11-2021 |
| | | | WO 2021112867 | A1 | 10-06-2021 |

EPO FORM P0459

For more details about this annex : see Official Journal of the European Patent Office, No. 12/82

**EP 4 302 693 A1**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 2017055934 A1